# EUROPEAN PATENT SPECIFICATION

(11) **EP 0 467 307 B1**
(45) Date of publication and mention of the grant of the patent: **09.10.1996**
(21) Application number: 91111870.1
(22) Date of filing: 16.07.1991
(51) Int. Cl.: G01N 33/00

(54) **Gas analyzer**
Gasanalysator
Analyseur de gaz

(30) Priority: 17.07.1990 JP 188254/90
(43) Date of publication of application: 22.01.1992
(73) Proprietor: HORIBA, LTD., Minami-ku Kyoto (JP)
(72) Inventor: Kada, Norio, Ibaraki-city, Osaka (JP); Noguchi, Naoki, Moriyama-city, Shiga-prefecture (JP); Imaki, Takao, Nagaokakyo-city, Kyoto (JP)
(74) Representative: TER MEER - MÜLLER - STEINMEISTER & PARTNER

(56) References cited:
- EP-A- 0 372 429
- FR-A- 2 249 332
- US-A- 4 316 382
- US-A- 4 432 226
- PATENT ABSTRACTS OF JAPAN, vol. 8, no. 202 (P-300)(1639), 14 September 1984

## Description

### BACKGROUND OF THE INVENTION

### Field of the invention

The present invention relates to a gas analyzer capable of simultaneously measuring concentrations of a plurality of ingredients contained in a sample gas or simultaneously measuring concentrations of the same ingredient contained in respective sample gases passing through a plurality of gas lines.

### Description of the Prior Art

In the case where for example NOₓ in air is measured, it is required to simultaneously put out a concentration of nitrogen monoxide (herein referred to as NO), nitrogen dioxide (hereinafter referred to as NO₂) and NOₓ, respectively.

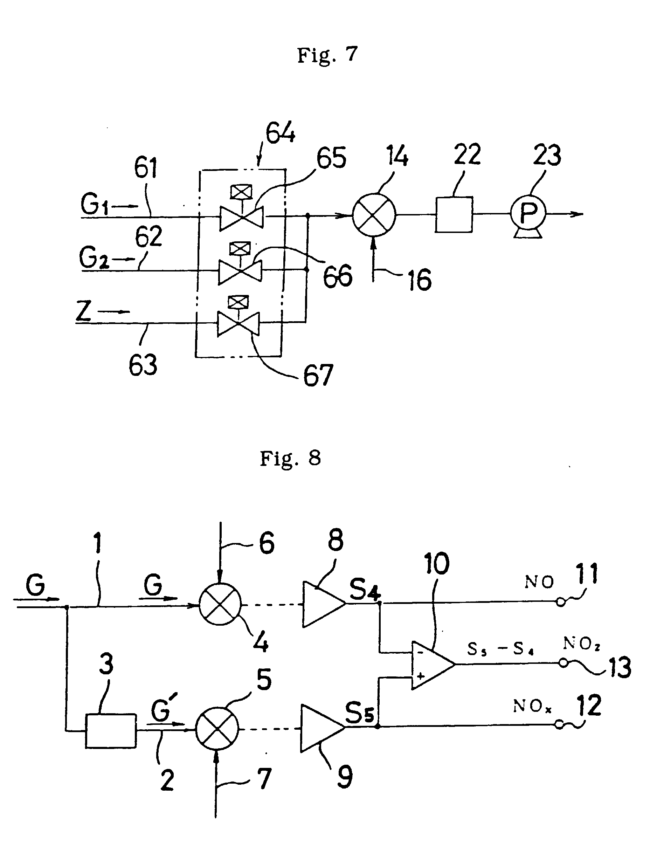

So, outputs have been simultaneously generated by a construction as shown in Fig. 8. That is to say, referring to Fig. 8, reference numeral 1 designates a first sample gas line through which a sample gas G (containing a plurality of ingredients including NO, NO₂ and NOₓ) from a sample gas source (not shown) flows as it is. Reference numeral 2 designates a second sample gas line branched from said first sample gas line 1 and provided with a convertor 3 for converting NO₂ contained in said sample gas G to NO. Through said second sample gas line 2 the sample gas G', which has been obtained by subjecting the sample gas G to some treatment, passes. Further, analytical portions 4, 5 of chemiluminescence detectors (hereinafter referred to as CLD's) are provided on the downstream sides of the gas lines 1 and 2, respectively. Reference numerals 6, 7 designate ozone lines for supplying said analytical portions 4, 5 with ozone, respectively, connected with ozonators (not shown). Reference numerals 8, 9 designate amplifiers for amplifying an output from the analytical portions 4, 5, respectively, reference numeral 10 designates a subtracter for obtaining a difference between output signals S₄, S₅ put out from the respective analytical portions 4, 5 through the respective amplifiers 8, 9, and reference numerals 11, 12, 13 designate output terminals.

In said CLD's having the above described construction, merely NO in the sample gases G, G' acts upon ozone when the analytical portions 4, 5 are supplied with the sample gases G, G' and ozone, so that the concentration of NO is measured by the analytical portion 4 and said signal S₄ corresponding to the concentration of NO is put out to said output terminal 11. In addition, the concentration of NOₓ is measured by the other analytical portion 5 and said signal S5 corresponding to the concentration of NOₓ is put out to said output terminal 12.

The concentration NOₓ - NO, in short a signal S₅ - S₄ corresponding to the concentration of NO2, is put out to said output terminal 13 by conducting a subtraction by means of said subtracter 10, whereby simultaneously obtaining the concentrations of NO, NO₂ and NOₓ in the sample gas G.

However, according to the above described construction, two analytical portions are required and thus costs increases. Further, said two analytical portions 4, 5 must be made equal to each other in measuring characteristic, such as change of sensitivity and responsiveness, in the case where a restrict measurement is required but such the matters are remarkably difficult to achieve. Accordingly, a great problem has occurred in that an accuracy in the measurement of NO₂ is lowered.

Such the problem has occurred in not only the above described CLD's but also in the case where a concentration of whole hydrocarbons, a concentration of methane and a concentration of hydrocarbons other than methane are simultaneously be measured by the use of a flame ionization detector (FID) and an infrared gas analyzer.

In addition, also in the case where the same ingredient contained in sample gases passing through two sample gas lines independent to each other is simultaneously measured, two analytical portions are required similarly to the above described conventional examples.

Accordingly, the above described problem has occurred similarly also in this case.

A gas analyzer known from FR-A-2 249 332 comprises a first sample gas line through which a sample gas passes as it is, a second sample gas line selectively branched from said first sample gas line and provided with a treatment portion for treating a specified ingredient, e.g. NO, in said sample gas and a zero gas line through which a zero gas passes. These gas lines are connected with a single analytical portion by means of a plurality of switchable valves controlled by a timer. The output of the analytical portion is connected to an evaluating circuit which further receives a control signal from the timer.

The different gases are successively introduced into the analytical portion in turn and the amplitudes of the output signal of the analytical portion are measured by means of the evaluating circuit.

A known gas chromatography (JP-A-5987361) comprises a first gas line for a carrier gas and a second gas line for a gas to be tested which are connected to a column via a valve system. The output of the column which separates the ingredients contained in a sample gas formed by introducing the gas to be tested into the carrier gas is connected to a detector the output of which is connected to a microcomputer. The microcomputer controls the detector and the valve system.

### SUMMARY OF THE INVENTION

The object of the present invention is to provide an inexpensive and highly accurate gas analyzer capable of simultaneously measuring concentrations of a plurality of ingredients contained in a sample gas. Another object of the present invention is to provide an inexpensive and highly accurate gas analyzer capable of simultaneously measuring concentration of the same ingredient contained in the respective sample gases passing through a plurality of gas lines.

This objects are achieved by a gas analyzer according to claims 1 and 2, respectively.

### BRIEF DESCRIPTION OF THE DRAWINGS

Preferred embodiments of the invention are shown in the drawings, in which
Fig. 1 is a schematic diagram showing a CLD to which the present invention is applied;
Fig. 2 is a diagram showing a relation between an opening and closing order of a control valve and an output from an analytical portion;
Fig. 3 is a diagram showing an example of a construction of a signal-treating portion;
Fig. 4 is a wave form diagram describing contents of a treatment in said signal-treating portion;
Fig. 5 is a schematic diagram showing a CLD according to another preferred embodiment;
Fig. 6 is a schematic diagram showing a CLD according to a further preferred embodiment;
Fig. 7 is a schematic diagram showing a still other embodiment according to the invention, and
Fig. 8 is a diagram showing a conventional example.

### DETAILED DESCRIPTION OF THE PREFERRED EMBODIMENTS

The preferred embodiments of the present invention will be below described with reference to the drawings.

In the following description. reference numerals same as those shown in Fig. 8 show members same as or corresponding to those in Fig. 8.

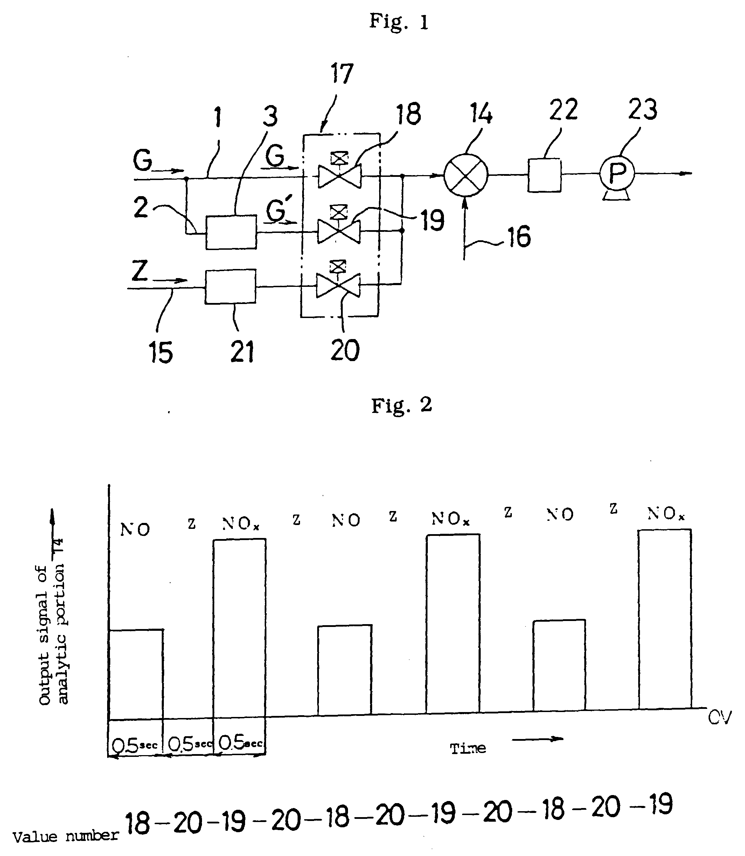

Figs. 1 to 4 show one preferred embodiment according to the invention. At first, Fig. 1 shows a rough construction of a CLD as a gas analyzer according to the present invention. Referring to Fig. 1, reference numeral 14 designates an analytical portion and a first sample gas line 1, through which a sample gas G from a sample gas source (not shown) passes as it is, a second sample gas line 2 branched from said first sample gas line 1 and provided with a treatment portion 3 (for example a convertor for converting NO₂ in said sample gas G to NO) for treating a specified ingredient in the sample gas G, through which a treated sample gas G' passes, and a zero gas line 15, through which a zero gas Z from a zero gas source (not shown) passes, are connected with said analytical portion 14 so that they may be parallel to each other. Further, an ozone line 16 for introducing ozone supplied from an ozonator (not shown) is connected with the analytical portion 14.

Reference numeral 17 designates a gas-controlling mechanism for introducing said gases G, G' and Z into the analytical portion 14 at an appointed cycle in the order described. Said gas-controlling mechanism 17 is provided in for example said gas lines 1, 2 and 15, respectively, and comprises electro-magnetic valves 18, 19, 20 controlledly opened and closed by means of a controller (not shown). The electro-magnetic valve 19 is provided on the downstream side of said convertor 3 and the electro-magnetic valve 20 is provided on the downstream side of a scrubber 21 for refining the zero gas Z. Reference numeral 22 designates an ozone-decomposer for decomposing ozone left in the gases discharged from the analytical portion 14. Reference numeral 23 designates a suction pump.

In said CLD having the above described construction, the sample gas G passing through the first sample gas line 1 and the sample gas G' passing through the second sample gas line 2 are adapted to be alternately introduced into the analytical portion 14 through the zero gas Z at an appointed cycle by controlledly opening and closing the electro-magnetic valves 18, 19, 20 at an appointed cycle under the condition that the analytical portion 14 is being supplied with ozone. That is to say, as shown in Fig. 2, the electro-magnetic valves 18, 19, 20 are opened for said appointed time by said appointed time (for example 0.5 seconds) in the order of the electro-magnetic valve 18 → the electro-magnetic valve 20 → the electro-magnetic valve 19 → the electro-magnetic valve 20 --- so that the analytical portion 14 may be supplied with the sample gases G, G' through the zero gas Z without fail. In the case where the analytical portion 14 is supplied with the gases G, G', Z in the above described manner, an output corresponding to NO, the zero gas and NOₓ, respectively, is obtained from the analytical portion 14, as shown in Fig. 2.

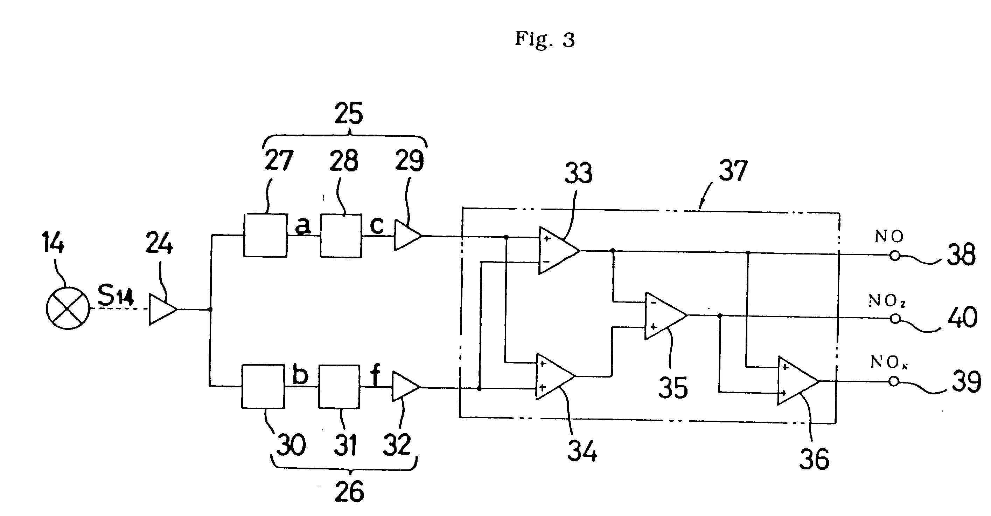

Fig. 3 shows an example of a construction of circuit for treating said outputs form the analytical portion 14. Referring to Fig. 3, reference numeral 24 designates a preamplifier for suitably amplifying the output S₁₄ from the analytical portion 14. And, two treating lines 25, 26 are provided on an output side of said preamplifier 24 for dividing the output S₁₄ into signals based on a plurality of modulation frequencies different to each other relating to said switch-cycle of the electromagnetic valves 18, 19, 20. That is to say, one treating line 25 is provided with a band pass filter 27 passing merely a signal of for example 1 Hz, a synchronous detecting circuit 28 for synchronously rectifying an output (a) from said band pass filter 27 at 1 Hz and a gain-regulating circuit 29 connected therewith in series. In addition, the other treating line 26 is provided with a band pass filter 30 passing merely a signal of for example 0.5 Hz, a synchronous detecting circuit 31 for synchronously rectifying an output (b) from said band pass filter 30 at 0.5 Hz and a gain-regulating circuit 32 connected therewith in series.

And, these treating lines 25, 26 are provided with an operating circuit 37 comprising a subtractor 33 for subtracting an output from the other treating line 26 from an output from the one treating line 25, an adder 34 for adding said outputs from said both treating lines 25, 26, a subtractor 35 for subtracting an output from said subtractor 33 from an output from said adder 34 and an adder 36 for adding outputs from the subtractor 33 and said subtractor 35 on the output side thereof. In addition, said operating circuit 37 is provided with an output terminal 38 for putting out the output from the subtractor 33 as it is, an output terminal 39 connected with an output side of said adder 36 and an output terminal 40 for putting out the output from the subtractor 35 as it is on an output side thereof.

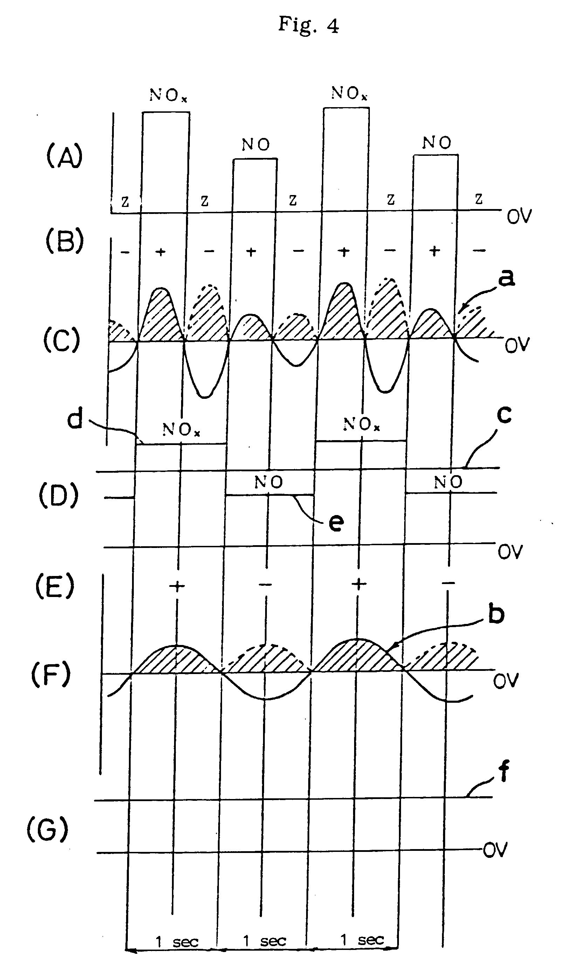

Next, an operation of the CLD having the above described construction is described with reference to also Fig. 4. Upon supplying the analytical portion 14 with the sample gas G passing through the first sample gas line 1 and the sample gas G' passing through the second sample gas line 2 through the zero gas Z passing through the zero gas line 15 by controlledly switching over the electro-magnetic valves 18, 19, 20 so that the time, for which they are opened, may amount to 0.5 seconds under the condition that the analytical portion 14 is being supplied with ozone, the output S₁₄ as shown in Fig. 3 and Fig. 4 (A) is put out from the analytical portion 14. This output S₁₄ is suitably amplified in the preamplifier 24 and then put into the treating line 25 and the treating line 26.

Upon putting the output S₁₄ in the band pass filter 27 of 1 Hz in the treating line 25, it is turned into a signal as shown by a full line in Fig. 4 (C). But, in this time, a synchronous signal is shown by positive and negative marks (-, +) in Fig. 4 (B), so that a signal (a) as hatched is put out after all. And, upon synchronously rectifying this signal (a) at 1 Hz in the synchronous detecting circuit 28, an output as shown by a full line (c) in Fig. 4 (D) is obtained and this output (c) is equal to an average {(NOₓ + NO)/2} of a concentration signal of NOₓ and a concentration signal of NO shown by (d) and (e) in Fig. 4 (D). The output (c) is put in the operating circuit 37 through the gain-regulating circuit 29.

In addition, upon putting the output S₁₄ in the band pass filter 30 of 0.5 Hz in the other treating line 26, it is turned into a signal as shown by a full line in Fig. 4 (F). But, in this time, a synchronous signal is shown by positive and negative marks (-, +) in Fig. 4 (E), so that a signal (b) as hatched is put out after all. And, upon synchronously rectifying this signal (b) at 0.5 Hz in the synchronous detecting circuit 31, an output as shown by a full line (f) in Fig. 4 (G) is obtained and this output (f) is equal to a half of a difference between said concentration signal of NOₓ and said concentration signal of NO {(NOₓ - NO)/2). The output (f) is put in the operating circuit 37 through the gain-regulating circuit 32.

Accordingly, upon putting said outputs (c), (f) in the subtractor 33 in the operating circuit 37 to make an appointed subtraction {(NOₓ + NO)/2 - (NOₓ - NO)/2}, a concentration signal of NO is obtained and put out to the output terminal 38. On the other hand, upon putting the outputs (c), (f) in the adder 34 to make an addition {(NOₓ + NO)/2 - (NOₓ - NO)/2}, the concentration signal of NOₓ is obtained. And, since the concentration signal of NO₂ is determined by subtracting the output from the subtractor 33 from the output from the subtractor 34 in the subtractor 35 to be put out to said output terminal 40 and the output from the subtractor 35 is added to the output from the subtractor 33 in said adder 36 to put out the concentration signal of NOₓ to said output terminal 39, the concentrations of NO, NO₂ and NOₓ in the sample gas G can be simultaneously obtained.

In addition, the construction of the operating circuit 37 can be variously modified. That is to say, the present Invention is not limited by this preferred embodiment.

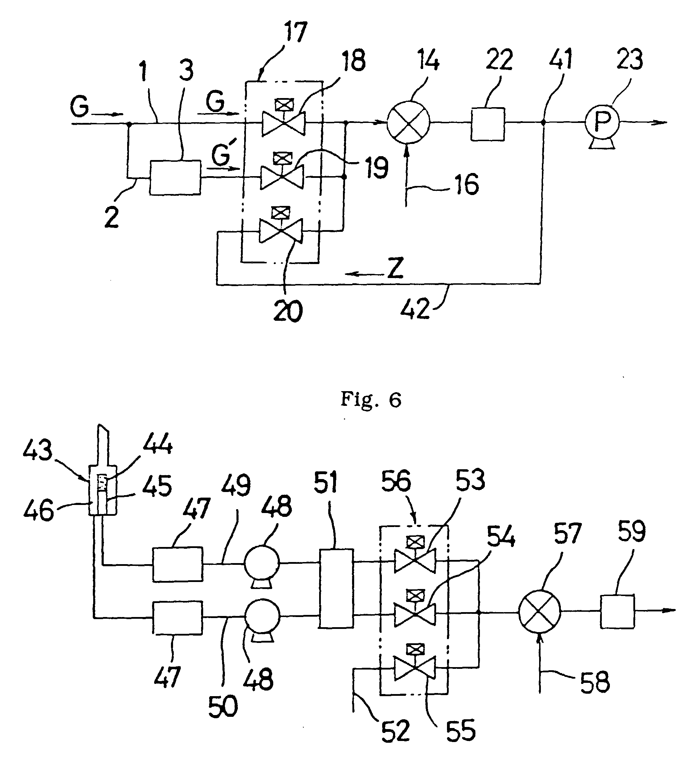

Since the ozone-decomposer 22 is provided on the downstream side of the analytical portion 14 in the above described preferred embodiment, ozone is decomposed even though it remains in the gas from the analytical portion 14. Accordingly, this gas, which passed through said ozone-decomposer 22, can be used as the zero gas. So, as shown in Fig. 5, a point 41 between the ozone-decomposer 22 and said suction pump 23 may be immediately connected with the upstream side of the electro-magnetic valve 20 by means of a piping 42 to use said piping 42 as the zero gas line. Thus, no zero gas source is required.

In addition, Fig. 6 is a schematic diagram showing a construction of a CLD in the case where ammonia (hereinafter referred to as NH₃) in a stack is measured. Referring to Fig. 6, reference numeral 43 designates a probe comprising a reaction tube 45 (corresponding to the above described treatment portion 3) including a denitrifying catalysis 44 and a reference tube 46 including no denitrifying catalysis 44 provided in a stack (not shown). Gas lines 49,50 provided with a convertor 47 for converting NOₓ and NH₃, respectively, to NO and a suction pump 48 in series are connected with said reaction tube 45 and said reference tube 46, respectively, and adapted to pass through an electronic cooler 51. In short, said gas lines 49, 50 are adapted to correspond to the first sample gas line 1 and the second sample gas line 2, respectively, in Fig. 1. Reference numeral 52 designates a zero gas line connected with a zero gas source (not shown).

And, the gas lines 49, 50 and said zero gas line 52 are connected with an analytic portion 57 in parallel to each other through respective electromagnetic valves 53, 54, 55 (these electro-magnetic valves 53, 54, 55 construct a gas-controlling mechanism 56 in the same manner as the above described electro-magnetic valves 18, 19, 20). In addition, reference numeral 58 designates an ozone line and reference numeral 59 designates an ozone-decomposer.

In said CLD having such the construction, a signal corresponding to a concentration obtained by subtracting a concentration of NH₃ from a concentration of NOₓ (hereinafter referred to as signal A) and a signal corresponding to said concentration of NOₓ (hereinafter referred to as signal B) are obtained from an analytical portion 57 by controlledly opening and closing the electro-magnetic valves 53, 54, 55 in the same manner as in the above described preferred embodiment.

And, in an operating circuit (not shown), said signal A is subtracted from said signal B to obtain said concentration of NH₃. In addition, a result obtained by subtracting the signal A from a result obtained by adding the signal B to the signal A is multiplied by 1/2 in order to obtain the concentration of NOₓ.

Fig. 7 shows a CLD according to a still further embodiment. Referring to Fig. 7, reference numerals 61, 62 designate a first sample gas line and a second sample gas line, through which a sample gas G₁ and a sample gas G₂ independent and different to each other passes, respectively. Said first sample gas line 61 and said second sample gas line 62 are connected with for example two furnaces (not shown) arranged in parallel, respectively, and with an analytical portion 14 in parallel to each other. In addition, reference numeral 63 designates a zero gas line, through which a zero gas Z from a zero gas source (not shown) passes, connected with said analytical portion 14 so as to be in parallel to the sample gas lines 61, 62. And, reference numeral 64 designates a gas-controlling mechanism for introducing said gas G₁, G₂ and Z into the analytical portion 14 in the order described at an appointed cycle comprising for example electro-magnetic valves 65, 66, 67 provided in the gas line 61, the gas line 62 and said gas line 63, respectively, and controlledly opened and closed by means of a control mechanism (not shown). In addition, in Fig. 7, reference numerals same as those in Fig. 1 designate members same as those in Fig. 1.

In said CLD having such the construction, concentration of the same ingredient, for example NO, contained in the sample gas G₁ passing through the first sample gas line 61 and the sample gas G₂ passing through the second sample gas line 62, respectively, can be simultaneously detected by a principle similar to that in the above described CLD shown in Fig. 1.

Although the above described preferred embodiments all relate to the CLD, the present invention is not limited by them but the present invention can be applied to also a FID and an infrared gas analyzer. For example, in the case where concentrations of whole hydrocarbons, methane and non-methane hydrocarbons are simultaneously measured, it is sufficient to use an oxidizer burning methane as the treatment portion 3.

In addition, it goes without saying that machines and tools other than the above described electro-magnetic valves may be used a said gas-controlling mechanism.

As above described, according to an embodiment as set forth in claim 1, the concentrations of a plurality of ingredients contained in the sample gas can be simultaneously measured. In addition, according to an embodiment as set forth in claim 2, the concentrations of the same ingredient contained in the sample gases passing through a plurality of sample gas lines can be simultaneously measured.

And, according to every one of the above described embodiments as set forth in claims 1 and 2, the gas analyzer can be simplified in construction and thus not only the gas analyzer can be made inexpensive so much as that but also a highly accurate measurement can be achieved.

## Claims

1. A gas analyzer, wherein a first sample gas line (1) through which a sample gas (G) containing a plurality of ingredients passes as it is, a second sample gas line (2) branched from said first sample gas line (1) and provided with a treatment portion (3) for treating a specified ingredient of said ingredients in said sample gas and a zero gas line (15), through which a zero gas (Z) passes, are connected with a single analytical portion (14) so that they may be parallel to each other; wherein the sample gas (G) passing through the first sample gas line (1) and the sample gas (G'), which has been treated, passing through said second sample gas line (2) are alternately introduced into said analytical portion (14) through said zero gas (Z) by means of a gas-controlling mechanism (17) to divide an output indicative for the concentrations of said ingredients from the analytical portion (14) at the time into signals based on a plurality of modulation frequencies different to each other relating to said switch over cycle, respectively, and these signals being added and subtracted to detect said concentrations of said ingredients contained in the sample gas.

2. A gas analyzer wherein a first sample gas line (61) and a second sample gas line (62) through which sample gases (G₁, G₂) containing the same ingredients independent and different to each other pass, respectively, and a zero gas line (63) through which a zero gas (Z) passes, are connected with a single analytical portion (14) so that they may be parallel to each other, said sample gas (61) passing through said first sample gas line (61) and said sample gas (G₂) passing through said second sample gas line (62) being alternately introduced into said analytical portion (14) through said zero gas (Z) by means of a gas-controlling mechanism (64) to divide an output indicative for the concentrations of said ingredient from the analytical portion (14) at that time into signals based on a plurality of modulation frequencies different to each other relating to said switch-over cycle, respectively, and these signals being added and subtracted to detect said concentrations of the same ingredient contained in the respective sample gases.

3. The gas analyzer as set forth in claim 1 or claim 2, wherein said output from the analytic portion (14) is divided into said signals based on a plurality of modulation frequencies different to each other relating to the switch-over cycle by means of two treating lines (25, 26) each comprising a band pass filter (27, 30), a synchronous detecting circuit (28, 31) and a gain-regulating circuit (29, 32).

4. The gas analyzer as claimed in one of the claims 1 to 3**, characterized in that** the open periods of the gas-controlling mechanism (17) for the gas passing through the first sample gas line, the gas passing through the second sample gas line and the zero gas are equal to each other.

5. The gas analyzer as claimed in claim 4, **characterized in that** the period of the modulation frequency of the band pass filter (27, 30) in the first treating line (25)/second treating line (26) is twice/fourth as high as the open period of the gas-controlling mechanism (17)(Fig. 4).

## Patentansprüche

1. Gasanalysator, bei dem eine erste Probengasleitung (1), durch die ein eine Mehrzahl von Bestandteilen enthaltendes Probengas (G), so wie es ist, hindurchströmt, eine zweite Probengasleitung (2), die von der ersten Probengasleitung (1) abzweigt und mit einem Behandlungsteil (3) zur Behandlung eines bestimmten Bestandteils der im Probengas enthaltenen Bestandteile versehen ist, und eine Nullgasleitung (15), durch die ein Nullgas (Z) hindurchströmt, mit einem einzigen Analyseteil (14) verbunden sind, derart, daß sie parallel zueinander liegen, wobei das durch die erste Probengasleitung (1) hindurchströmende Probengas (G) und das durch die zweite Probengasleitung (2) hindurchströmende behandelte Probengas (G') abwechselnd dem Analyseteil (14), getrennt durch das Nullgas (Z), zugeführt werden, und zwar mittels eines Gassteuermechanismus (17), um einen die Konzentrationen der Bestandteile angebenden Ausgang vom Analyseteil (14) in Signale zu unterteilen, und zwar auf der Grundlage einer Mehrzahl von untereinander verschiedenen Modulationsfrequenzen, die jeweils Bezug zum Umschaltzyklus haben, und wobei diese Signale addiert und subtrahiert werden, um die Konzentrationen der im Probengas enthaltenen Bestandteile zu detektieren.

2. Gasanalysator, bei dem eine erste Probengasleitung (61) und eine zweite Probengasleitung (62), durch die dieselben Bestandteile enthaltene Probengase (G₁, G₂), die unabhängig und verschieden voneinander sind, hindurchströmen, und eine Nullgasleitung (63), durch die ein Nullgas (Z) hindurchströmt, mit einem einzigen Analyseteil (14) so verbunden sind, daß sie parallel zueinander liegen, wobei das durch die erste Probengasleitung (61) hindurchströmende Probengas (G₁) und das durch die zweite Probengasleitung (62) hindurchströmende Probengas (G₂) abwechselnd dem Analyseteil (14), getrennt durch das Nullgas (Z), zugeführt werden, und zwar unter Steuerung eines Gassteuermechanismus (64), um einen die Konzentrationen der Bestandteile angebenden Ausgang vom Analyseteil (14) zu dieser Zeit in Signale zu unterteilen, und zwar auf der Grundlage einer Mehrzahl von sich unterscheidenden Modulationsfrequenzen, die jeweils einen Bezug zum Umschaltzyklus haben, und wobei diese Signale addiert und subtrahiert werden, um die Konzentrationen desselben in den jeweiligen Probengasen vorhandenen Bestandteils zu detektieren.

3. Gasanalysator nach Anspruch 1 oder 2, **dadurch gekennzeichnet**, daß der Ausgang des Analyseteils (14) unter Verwendung von zwei Behandlungsleitungen (25, 26) in die beiden Signale unterteilt wird, und zwar basierend auf einer Mehrzahl von sich unterscheidenden Modulationsfrequenzen, die einen Bezug zum Umschaltzyklus haben, wobei jede der Behandlungsleitungen (25, 26) einen Bandpassfilter (27, 30), eine Synchrondetektorschaltung (28, 31) und eine Verstärkungsregulierschaltung (29, 32) aufweisen.

4. Gasanalysator nach einem der Ansprüche 1 bis 3, **dadurch gekennzeichnet**, daß die Öffnungsperioden des Gassteuermechanismus (17) für das die erste Probengasleitung durchströmende Gas, für das die zweite Probengasleitung durchströmende Gas und für das Nullgas einander gleich sind.

5. Gasanalysator nach Anspruch 4, **dadurch gekennzeichnet**, daß die Periode der Modulationsfrequenz des Bandpassfilters (27, 30) in der ersten Behandlungsleitung (25)/zweiten Behandlungsleitung (26) zweimal/viermal so groß ist wie die Öffnungsperiode des Gassteuermechanismus (17) (Fig.4).

## Revendications

1. Un analyseur de gaz, dans lequel un premier conduit de gaz échantillon (1) parcouru par un gaz échantillon (G) contenant une pluralité d'ingrédients ou composants, un deuxième conduit de gaz échantillon (2) branché en dérivation dudit premier conduit de gaz échantillon (1) et muni d'une partie de traitement (3) pour traiter un ingrédient spécifique desdits ingrédients dans ledit gaz échantillon, ainsi qu'un conduit de gaz à zéro ingrédient (15) parcouru par un gaz témoin ou à zéro ingrédient (Z), sont reliés à une partie d'analyse unique (14) de façon à ce qu'ils puissent être parallèles l'un à l'autre; dans lequel le gaz échantillon traversant le premier conduit de gaz échantillon (1) et le gaz échantillon (G') qui a été traité, traversant ledit deuxième conduit de gaz échantillon (2) sont alternativement introduits dans ladite partie d'analyse (14) à travers ledit gaz à zéro ingrédient (Z) au moyen d'un mécanisme de contrôle de gaz (17) pour diviser un signal de sortie indicatif des concentrations desdits ingrédients obtenues de la partie d'analyse (14) de façon temporelle, pour obtenir des signaux basés sur une pluralité de fréquences de modulation respectivement différentes l'une de l'autre par rapport audit cycle de basculement, lesdits signaux étant additionnés et soustraits afin de mesurer lesdites concentrations desdits ingrédients contenus dans le gaz échantillon.

2. Un analyseur de gaz, dans lequel un premier conduit de gaz échantillon (61) et un deuxième conduit de gaz échantillon (62) parcouru par des gaz échantillon (G₁, G₂) contenant respectivement les mêmes ingrédients mais de façon indépendante et différente l'un de l'autre, ainsi qu'un conduit de gaz à zéro ingrédient (63) parcouru par un gaz à zéro ingrédient (Z), sont reliés à une partie d'analyse unique (14) de façon à ce qu'ils puissent être parallèles l'un à l'autre, ledit gaz échantillon (G1) traversant ledit premier conduit de gaz échantillon (61) et ledit gaz échantillon (G2) traversant ledit deuxième conduit de gaz échantillon (62) alternativement étant introduits, dans ladite partie d'analyse (14) à travers ledit gaz à zéro ingrédient (Z) au moyen d'un mécanisme de contrôle de gaz (64) pour diviser un signal de sortie indicatif des concentrations desdits ingrédients obtenues de la partie d'analyse (14) de façon temporelle, pour obtenir des signaux basés sur une pluralité de fréquences de modulation respectivement différentes l'une de l'autre par rapport audit cycle de basculement, lesdits signaux étant additionnés et soustraits afin de mesurer lesdites concentrations du même ingrédient conteu dans les gaz échantillons respectifs.

3. L'analyseur de gaz selon la revendication 1 ou 2, dans lequel ledit signal de sortie de la partie d'analyse (14) est divisé en lesdits signaux basés sur une pluralité de fréquences de modulation différente l'une de l'autre par rapport au cycle de basculement, au moyen de deux lignes moteur de traitement (25, 26) comprenant chacune un filtre passe-bande (27, 30), un circuit de détection synchone (28, 31) et un circuit de réglage de gain (29, 32).

4. L'analyseur de gaz selon l'une des revendications 1 à 3, caractérisé en ce que les durées d'ouverture du mécanisme de contrôle de gaz (17) pour le gaz traversant le premier conduit de gaz échantillon, le gaz traversant le deuxième conduit de gaz échantillon et le gaz à zéro ingrédient sont égales l'une à l'autre.

5. L'analyseur de gaz selon la revendication 4, caractérisé en ce que la période de la fréquence de modulation du filtre passe-bande (27, 30) de la première (25)/deuxième (26) ligne de traitement est deux fois/quatre fois plus grande que la durée d'ouverture du mécanisme de contrôle de gaz (17) (figure 4)
